# EUROPEAN PATENT SPECIFICATION

(11) **EP 1 664 168 B1**
(45) Date of publication and mention of the grant of the patent: **03.12.2008**
(21) Application number: 04782412.3
(22) Date of filing: 27.08.2004
(51) Int. Cl.: C08J 9/00, C08K 5/00

(54) **HYDROGEL POROGENS FOR FABRICATING BIODEGRADABLE SCAFFOLDS**
HYDROGEL-PORENBILDNER ZUR HERSTELLUNG VON BIOLOGISCH ABBAUBAREN GERÜSTEN
AGENTS POROGENES A BASE D'HYDROGEL PERMETTANT DE FABRIQUER DES OSSATURES BIODEGRADABLES

(30) Priority: 29.08.2003 US 498832 P
(43) Date of publication of application: 07.06.2006
(73) Proprietor: Mayo Foundation for Medical Education and Research, Rochester, MN 55905 (US)
(72) Inventor: JABBARI, Esmaiel, Rochester, MN 55901 (US); YASZEMSKI, Michael, J., Rochester, MN 55902 (US); CURRIER, Bradford, L., Rochester, MN 55902 (US); LU, Lichun, Rochester, MN 55901 (US)
(74) Representative: Englaender, Klaus
(86) International application number: PCT/US2004/027926
(87) International publication number: WO 2005/020849

(56) References cited:
- EP-A- 0 000 507
- US-B1- 6 271 278
- US-B1- 6 395 300
- PATENT ABSTRACTS OF JAPAN vol. 2003, no. 11, 5 November 2003 (2003-11-05) & JP 2003 182208 A (TOYOBO CO LTD), 3 July 2003 (2003-07-03)
- PATENT ABSTRACTS OF JAPAN vol. 2003, no. 08, 6 August 2003 (2003-08-06) & JP 2003 103905 A (MITSUBISHI PAPER MILLS LTD), 9 April 2003 (2003-04-09)

## Description

### CROSS-REFERENCES TO RELATED APPLICATIONS

This application claims priority from United States Provisional Patent Application No. 60/498,832 filed August 29, 2003.

### STATEMENT REGARDING FEDERALLY SPONSORED RESEARCH

This work was supported by the National Institutes of Health through grant number R01-AR45871-02.

### BACKGROUND OF THE INVENTION

### 1. Field of the Invention

This invention relates to the synthesis of biodegradable, biocompatible scaffolds for tissue engineering applications and more particularly to hydrogel porogens for fabricating biodegradable scaffolds.

### 2. Description of the Related Art

In the field of tissue engineering, biodegradable polymeric biomaterials can serve as a scaffold to provide mechanical support and a matrix for the ingrowth of new tissue. As new tissue forms in and/or on the scaffold, the biomaterial degrades until it is entirely dissolved. The degradation products are eliminated through the body's natural pathways, such as metabolic processes. One example of the use of such biomaterials is as a temporary bone replacement to replace or reconstruct all or a portion of a living bone. The bone tissue grows back into the pores of the polymeric implant and will gradually replace the entire implant as the polymeric implant itself is gradually degraded in the *in vivo* environment.

For minimally invasive applications, biodegradable scaffolds that can be injected and crosslinked in situ are attractive candidates to treat bone defects after skeletal trauma. Several techniques are known for fabricating porous polymeric scaffolds for bone tissue engineering applications, and significant progress has been made in the past decade to fabricate polymer scaffolds with controlled morphology and microarchitecture. However, most of these techniques, such as fiber bonding, compression molding, extrusion, freeze-drying, phase separation, and various solid freeform fabrication methods are not compatible for use with injectable materials.

Traditionally, two methods are used to fabricate porous injectable scaffolds. First, a gas foaming technique is based on the reaction between an acid (e.g., citric acid or L-ascorbic acid) and a base (e.g., sodium or calcium bicarbonate) to produce carbon dioxide which causes foaming and produces porosity within the scaffold. An example of this type of foaming technique can be found in U.S. Patent No. 6,562,374. Second, a salt leaching technique represents simple mixing of salt crystals (e.g., sodium chloride) of known size with the polymer matrix of the composite scaffold. Once exposed to liquid, salt crystals are leached out creating a system of mutually interconnected pores. Examples of this type of particle leaching technique can be found in U.S. Patent Nos. 6,436,426, 6,379,962 and 5,514,378. A combination of these two techniques is also proposed in U.S. Patent No. 6,281,256.

Both techniques have inherent limitations which prevent them from being considered as optimal porosity forming methods. Design criteria for selection of a porogen for injectable scaffolds include mechanical strength of the scaffold following porogen leach-out, toxicity of the leached porogen, and rheological properties of the porogen/polymer composite prior to injection, and uniform distribution of the porogen in the composite scaffold. There are many issues associated with using sodium chloride salt crystals as porogen. For example, prior salt leaching work demonstrates a maximum porosity (about 75%) above which the solid salt porogen particles do not flow in a homogeneous manner with the polymerizing scaffold during injection. (See, Hedberg et al., Controlled Release of an Osteogenic Peptide from Injectable Biodegradable Polymeric Composites, J. Control. Release, 84, 137-150, 2002.) Due to the limited amount of porogen that can be incorporated, it is often difficult to achieve the high porosity needed to induce tissue ingrowth and minimize diffusion limitations. In addition, once the scaffold has been injected, the process of porogen diffusion out of the scaffold needs to occur prior to the scaffold pores being filled with physiologic fluids. Massive salt leaching, occurring immediately after exposure of the implant to water, can result in a highly hypertonic environment with detrimental effect on cells surrounding the implant. The foaming technique addresses some issues associated with salt-leaching but introduces new issues such as a wide range of pore sizes, non-uniform distribution of pores, and low interconnectivity between the pores. Both of these methods are based on the assumption that the porogen must be replaced by the physiologic fluid surrounding the scaffold to allow tissue in-growth.

Thus, there is a need for an improved porogen for an injectable composition that is used to fabricate porous scaffolds.

### SUMMARY OF THE INVENTION

In this invention, hydrogel microparticles with entrapped liquid are used as the porogen to reproducibly form interconnected pore networks in a scaffold. For example, EP 0000507 teaches the formation of such hydrogel microparticles. Hydrogel microparticles can be incorporated into an injectable paste including a biodegradable unsaturated polymer and a crosslinking agent, and instead of leaching out, the microparticles swell during mixing and injection to retain water in the pores. The advantages of hydrogel microparticles as porogen include better rheologic properties during injection, elimination of the porogen leaching step, no detrimental effect of porogen on cells surrounding the implant, and the ability to load and deliver either cells, bioactive molecules, or both with the hydrogel porogen at the time of the scaffold injection at the bone repair site. Moreover, when hydrogel microparticles are used as porogen, they provide a permissive environment for tissue in-growth.

In one aspect, the invention provides a composition for fabricating a porous scaffold. The composition includes a biodegradable unsaturated polymer, a crosslinking agent, and biodegradable hydrogel microparticles, wherein the hydrogel microparticles include entrapped water. Preferably, the composition is injectable. The composition or the microparticles may further include one or more bioactive agents. Optionally, the biodegradable unsaturated polymer is self-crosslinkable. Example biodegradable unsaturated polymers include poly(propylene fumarate), poly(ε-caprolactone-fumarate), and mixtures and copolymers thereof. The crosslinking agent may be a free radical initiator, or may include a free radical initiator and a monomer capable of addition polymerization.

In one form, the hydrogel microparticles include uncrosslinked or crosslinked collagen, an uncrosslinked or crosslinked collagen derivative or mixtures thereof. In another form, the hydrogel microparticles include an uncrosslinked or crosslinked synthetic biodegradable polymer such as uncrosslinked or crosslinked oligo(poly(ethylene glycol) fumarate). Preferably, the hydrogel microparticles have a water content from about 20% to about 99% by volume and more preferably a water content from 70% to 95%. In one form, the hydrogel microparticles have diameters in the range of 1 to 1000 micrometers.

In another aspect, the invention provides a scaffold for tissue regeneration. The scaffold is prepared by allowing any of the compositions according to the invention to crosslink in a cavity or a mold. In one form, the hydrogel microparticles comprise greater than 49% by volume and up to 99% by volume of the scaffold. The hydrogel microparticles may include physiologic fluid that replaces the entrapped water after the scaffold is placed into or formed in a cavity in an animal or human body.

In yet another aspect, the invention provides a method for fabricating a scaffold for tissue regeneration. The method includes the step of allowing any of the compositions according to the invention to crosslink in a cavity or a mold. In one application, the cavity is in an animal or human body. The hydrogel microparticles may include physiologic fluid that replaces the entrapped water after the composition crosslinks in the cavity.

Thus, the invention provides for the use of hydrogel microparticles as a porogen to form interconnected porous scaffolds. The use of hydrogel microparticles as the porogen provides a permissive environment for tissue ingrowth within the scaffold without the need for the process of porogen diffusion out of the scaffold prior to the scaffold pores being filled with physiologic fluid.

These and other features, aspects, and advantages of the present invention will become better understood upon consideration of the following detailed description, drawings, and appended claims.

### BRIEF DESCRIPTION OF THE DRAWINGS

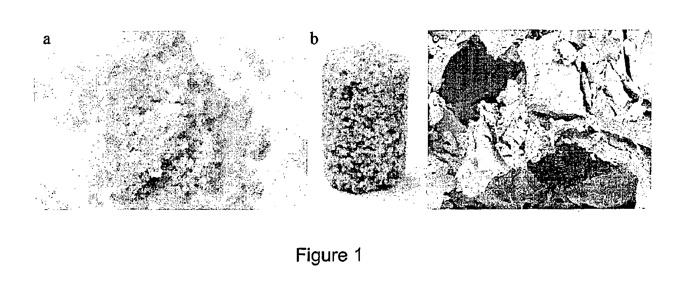

Figure 1a shows an image of gelatin microspheres with average size of 500 µm and equilibrium water content of 85%.

Figure 1b shows a poly(propylene fumarate) composite scaffold fabricated with 80% by volume gelatin microspheres.

Figure 1c is an SEM micrograph which shows the pores left behind in the poly(propylene fumarate) scaffold after the degradation of the gelatin microspheres in phosphate buffered saline for 5 days.

### DETAILED DESCRIPTION OF THE INVENTION

This invention describes a process for the fabrication of porous biodegradable scaffolds. In this process, hydrogel microparticles are used as the porogen to make biodegradable porous polymeric scaffolds for applications in tissue engineering. In this process, super-absorbent semi-solid hydrogel microparticles, that swell in aqueous environment but do not flow as liquids due to crosslinking and have a defined semi-solid shape due to their crosslinked molecular structure, are mixed with a biodegradable unsaturated polymer by physical blending. Next, appropriate amounts of a crosslinking agent are added and mixed with the hydrogel microparticles/polymer mixture. The polymerizing mixture is transferred to a mold and it is crosslinked by the action of the crosslinking agent. The polymerizing mixture can also be injected or inserted into a body cavity such as a skeletal defect of any size or shape.

If the biodegradable unsaturated polymer is capable of self-crosslinking (i.e., the polymer is "self-crosslinkable" polymer such as poly(ε-caprolactone-fumarate)), the crosslinking agent need only be a free radical initiator. The crosslinking can take place by way of the free radical initiator that adds to the carbon-carbon double bonds of the unsaturated polymer to produce carbon radicals that further react with other carbon radicals formed in the unsaturated polymer. Example free radical initiators include, without limitation, benzoyl peroxide, acetyl peroxide, azobisisobutyronitrile, and ascorbic acid. If the biodegradable unsaturated polymer is not capable of self-crosslinking or crosslinks slowly, the crosslinking agent may include a free radical initiator and a monomer capable of addition polymerization, such as 1-vinyl-2-pyrrolidinone, methylmethacrylate, poly(propylene fumarate)-diacrylate (PPFDA), poly(ethylene glycol)-diacrylate (PEGDA), or poly(ethylene glycol)-dimethacrylate (PEGDMA).

One or more bioactive agents can also be added to the hydrogel microparticles/polymer mixture or entrapped in the hydrogel microparticles. The bioactive agent or bioactive agents are selected depending on the physiological effect desired. A "bioactive agent" as used herein includes, without limitation, physiologically or pharmacologically active substances that act locally or systemically in the body. A bioactive agent is a substance used for the treatment, prevention, diagnosis, cure or mitigation of disease or illness, or a substance which affects the structure or function of the body or which becomes biologically active or more active after it has been placed in a predetermined physiological environment. Bioactive agents include, without limitation, enzymes, organic catalysts, ribozymes, organometallics, proteins, glycoproteins, peptides, polyamino acids, antibodies, nucleic acids, steroidal molecules, antibiotics, antimycotics, cytokines, growth factors, carbohydrates, oleophobics, lipids, extracellular matrix and/or its individual components, pharmaceuticals, and therapeutics.

An accelerator, such as N,N dimethyl toluidine or tetramethylethylenediamine, can also be added to the hydrogel microparticles/polymer mixture. Inorganic fillers, such as tricalcium phosphate and hydroxyapatite, can also be added. The amounts of each optional additive may be varied according to the desired characteristics of the final scaffold.

A scaffold formed using the hydrogel microparticle/polymer mixture can be used directly for skeletal defects without the need to leach out the hydrogel porogen. In this process, the liquid (i.e., water) content of the crosslinked hydrogel microparticles can be anywhere from 20% to 99% by volume. The hydrogel microparticles can be loaded in the scaffold in amounts greater than 49% by volume and up to 99% by volume of the scaffold. The hydrogel microparticles can be selected from natural and synthetic hydrogels. For example, the hydrogel microparticles may be based on natural biodegradable polymers such as gelatin or collagen or can be based on synthetic biodegradable polymers such as oligo(poly(ethylene glycol) fumarate) (OPF). The matrix polymer can be any biodegradable polymer containing unsaturated carbon-carbon double bonds. Non-limiting examples include poly(propylene fumarate) (PPF) or poly(ε-caprolactone-fumarate). The scaffold can be preformed in a mold before implantation or it can be injected and crosslinked in situ in a body cavity.

A typical example procedure for the fabrication of gelatin hydrogel microparticles is as follows. A solution of porcine gelatin powder in distilled deionized water is prepared by heating to 95 degrees centigrade while mixing. Next, the gelatin solution is added dropwise from a syringe into mineral oil containing varying concentrations of dithiobis(succinimidylpropionate) (DSP) as the crosslinking agent. The mineral oil is cooled to 10 degrees centigrade while agitating with a magnetic stirrer. Gelatin microparticles will form due to agitation and harden physically by cooling and chemically by crosslinking. The microparticles are filtered and washed with phosphate buffered saline (PBS) and stored at -20 degrees centigrade until use. The concentration of gelatin in the aqueous phase and the concentration of DSP in the mineral phase will control the equilibrium water content of microparticles. The size of the microparticles is controlled by the agitation rate and the needle size used for dropwise addition of gelatin solution to the mineral oil phase. A Coulter counter is used to determine the size distribution of microparticles and their surface morphology is examined with scanning electron microscopy. The water content and degree of crosslinking is determined by equilibrium swelling experiments in PBS at 37 degrees centigrade.

The same procedure for fabrication of gelatin microparticles can be used to fabricate collagen microparticles except that the collagen is dispersed in distilled deionized water at 4 degrees centigrade by homogenization.

A typical example procedure for fabrication of the synthetic and biodegradable oligo(poly(ethylene glycol) fumarate) microparticles from oligo(poly(ethylene glycol) fumarate) macromer is as follows. The size of the oligo(poly(ethylene glycol) fumarate) microparticles can be controlled by the molecular weight of poly(ethylene glycol) of the oligo(poly(ethylene glycol) fumarate) macromer, extent of crosslinking, and the water content of the aqueous phase before crosslinking. Ammonium persulfate (APS), tetramethyethylenediamine (TMED), and methylene bisacrylamide (BISAM) are used as the initiator, accelerator, and crosslinking agent, respectively. The aqueous hydrogel phase is prepared by mixing 1 gram of OPF, 0.1 grams MBA, 0.4 ml. of 0.3 M APS in PBS, and 0.4 ml. of 0.3 M TMED. The aqueous phase is added dropwise with stirring to mineral oil containing 1.5 and 0.5% by weight sorbitan monooleate and polyoxyethylene sorbitan monooleate, respectively. The crosslinking reaction is carried out at 40 degrees centigrade with stirring and under nitrogen atmosphere for 20 minutes. After the reaction, OPF microspheres are washed with hexane to remove the mineral oil, residual hexane is removed under reduced pressure, and stored at -20 degrees centigrade until use.

The following example procedure may be used to fabricate scaffolds with hydrogel microparticles as the porogen. Scaffolds are prepared by crosslinking poly(propylene fumarate) (PPF) and a cross-linking monomer, such as 1-vinyl-2-pyrrolidinone (NVP), by free radical polymerization with hydrogel microparticles as the porogen. Benzoyl peroxide and dimethyl toluidine are used as the free radical initiator and the accelerator, respectively. Different amounts of hydrogel microparticles are added to the PPF/NVP mixture to fabricate scaffolds with volume porosities of 50% up to 95% by volume. First, 0.71 grams of PPF with 0.29 grams of NVP, corresponding to 40% NVP per gram of PPF, are mixed in a scintillation vial for 2 hours at 37 degrees centigrade. Then, to the mixture, 5 grams of hydrogel microparticles as porogen is added, corresponding to 75% by volume porosity. Next, 0.05 ml. of initiator solution (50 mg. of benzoyl peroxide in 0.25 ml. of 1-vinyl-2-pyrrolidinone) and 0.02 ml. of accelerator solution (0.02 ml. of dimethyltoluidine in 0.98 ml. of methylene chloride) are added and mixed thoroughly. The resulting paste is transferred into a 5 mm. x 18 mm. Teflon® mold and pressed manually to maximize packing. The mold is placed in a convection oven for 1 hour at 40 degrees centigrade to facilitate crosslinking. After crosslinking, the mold is cooled to ambient temperature, scaffolds are removed from the mold, and cylindrical specimens with desired diameter and length are cut with a Isomet low speed saw. The scaffold can be implanted directly after sterilization without the need for leaching out the porogen or without the need for drying.

The same procedure can be used to prepare an injectable paste except that the resulting paste is injected into a cavity instead of transferring into a mold.

As used herein, a "biocompatible" material is one which stimulates only a mild, often transient, implantation response, as opposed to a severe or escalating response. As used herein, a "biodegradable" material is one which decomposes under normal *in vivo* physiological conditions into components which can be metabolized or excreted. By "injectable", we mean the copolymer may be delivered to a site by way of a syringe. By "self-crosslinkable", we mean the functional groups of a first polymer having specific repeating groups may crosslink with the functional groups of a second polymer having the same repeating groups without a crosslinking compound that forms crosslinking bridges between the repeating groups of the first polymer and the repeating groups of the second polymer. As used herein, a "collagen derivative" is a substance which is obtained by chemically or physically altering naturally occurring collagen. For example, one preferred collagen derivative, gelatin, can be obtained by boiling collagen in water.

### Example

The following Example has been presented in order to further illustrate the invention and is not intended to limit the invention in any way.

Gelatin microspheres were prepared by a method developed previously (see Payne et al., Development of an Injectable, In Situ Crosslinkable, Degradable Polymeric Carrier for Osteogenic Cell Populations. Part 1. Encapsulation of Marrow Stromal Osteoblasts in Surface Crosslinked Gelatin Microparticles, Biomaterials, 23, 4359-4371, 2002). Briefly, a solution of porcine gelatin powder in distilled deionized water (ddH₂O), prepared by heating to 95°C, was added dropwise into mineral oil containing varying concentrations of dithiobis(succinimidylpropionate) (DSP) as the crosslinking agent. Gelatin microspheres formed and hardened by crosslinking. The microparticles were filtered and washed with phosphate buffered saline (PBS). The concentration of gelatin in the aqueous phase and that of DSP in the mineral phase controlled the equilibrium water content of the microparticles. The agitation rate controlled the size of the microparticles. The size distribution of the microparticles and their surface morphology were determined with a Coulter counter and by SEM, respectively. The water content and degree of crosslinking was determined by equilibrium swelling experiments in PBS at 37°C. The same procedure was used to fabricate collagen microspheres except that the collagen was dispersed in ddH₂O at 4°C by homogenization (see Park et al., Characterization of porous collagen/hyaluronic acid scaffold modified by 1-ethyl-3-(3-dimethylaminopropyl) carbodiimide cross-linking, Biomaterials, 23, 1205-1212, 2002).

Synthetic Oligo(poly(ethylene glycol)-fumarate) (OPF) microparticles were synthesized by condensation polymerization of fumaryl chloride (FC) with poly(ethylene glycol) (PEG) (see Jo et al., Synthesis and Characterization of Oligo(Poly(Ethylene Glycol) Fumarate) Macromer, Macromolecules, 34, 2839-2844, 2001). The following procedure was used to synthesize OPF microparticles from OPF macromer by suspension free radical polymerization (see Jabbari et al., Controlled Release of Plasmid DNA from Biodegradable Oligo( poly(ethylene glycol) fumarate) Hydrogel Microspheres, Proc. Ann. Meet. AlChE, abstract 305a, p. 141, 2002). The size of the OPF microparticles is controlled by the molecular weight of PEG, extent of crosslinking, and the water content of the aqueous phase. Ammonium persulfate (APS), tetramethyethylenediamine (TMED), and methylene bisacrylamide (MBA) were used as the initiator, accelerator, and crosslinking agent, respectively. The aqueous phase was added dropwise with stirring to mineral oil containing 1.5 and 0.5% by weight sorbitan monooleate and polyoxyethylene sorbitanmonooleate, respectively. The crosslinking reaction was carried out at 40°C with stirring and under nitrogen atmosphere for 20 minutes.

A scaffold was prepared by free radical polymerization of poly(propylene fumarate) (PPF) with gelatin, collagen or OPF microparticles as the porogen. Benzoyl peroxide and dimethyl toluidine were used as the free radical initiator and accelerator, respectively. In a typical procedure, 0.9 grams of PPF and 0.36 grams of 1-vinyl-2-pyrrolidinone (NVP) as the crosslinking agent were mixed with 5 grams of porogen, corresponding to 80% by volume porosity. Then, 50 µl of initiator solution and 40 µl of accelerator solution were added. The resulting paste was transferred into a 5 mm. x 18 mm. Teflon® mold and allowed to crosslink for 1 hour. The crosslinking process was characterized with rheometry. The pore structure and pore morphology were examined by scanning electron microscopy.

Results: Figure 1 a shows an image of the gelatin microspheres with average size of 500 µm and equilibrium water content of 85%. Natural and synthetic hydrogel microspheres with diameters in the range of 300-700 µm and water content of 70 to 95% were synthesized by the above procedures. The degradation time can be varied from a few days for gelatin and collagen to a few months for synthetic OPF microspheres. A PPF composite scaffold fabricated with 80% by volume gelatin microspheres is shown in Figure 1 b. The SEM micrograph in Figure 1c shows the pores left behind in the PPF scaffold after the degradation of gelatin microspheres in PBS for 5 days. These results show that at least one of the limitations of salt crystals as the porogen can be overcome by the use of hydrogels as the porogen in fabrication of interconnected porous scaffolds.

Although the present invention has been described in considerable detail with reference to certain embodiments, one skilled in the art will appreciate that the present invention can be practiced by other than the described embodiments, which have been presented for purposes of illustration and not of limitation. Therefore, the scope of the appended claims should not be limited to the description of the embodiments contained herein.

## Claims

1. A composition for fabricating a porous scaffold, the composition comprising:
a biodegradable unsaturated polymer;
a crosslinking agent; and
a biodegradable hydrogel microparticles porogen,
wherein the hydrogel microparticles include entrapped water.

2. The composition of claim 1 wherein:
the biodegradable unsaturated polymer is self-crosslinkable.

3. The composition of claim 1 wherein:
the crosslinking agent is a free radical initiator.

4. The composition of claim 1 wherein:
the crosslinking agent comprises a free radical initiator and a monomer capable of addition polymerization.

5. The composition of claim 1 wherein:
the hydrogel microparticles comprise collagen, a collagen derivative or mixtures thereof.

6. The composition of claim 1 wherein:
the hydrogel microparticles comprise crosslinked collagen, a crosslinked collagen derivative or mixtures thereof.

7. The composition of claim 1 wherein:
the hydrogel microparticles comprise a synthetic biodegradable polymer.

8. The composition of claim 1 wherein:
the hydrogel microparticles comprise a crosslinked synthetic biodegradable polymer.

9. The composition of claim 1 wherein:
the hydrogel microparticles comprise oligo(poly(ethylene glycol) fumarate).

10. The composition of claim 1 wherein:
the hydrogel microparticles comprise crosslinked oligo(poly(ethylene glycol) fumarate).

11. The composition of claim 1 wherein:
the hydrogel microparticles have a water content from about 20% to about 99% by volume.

12. The composition of claim 1 wherein:
the composition is injectable.

13. The composition of claim 1 wherein:
the biodegradable unsaturated polymer is selected from poly(propylene fumarate), poly(ε-caprolactone-fumarate), and mixtures and copolymers thereof.

14. The composition of claim 1 wherein:
the biodegradable unsaturated polymer comprises poly(propylene fumarate).

15. The composition of claim 1 wherein:
the hydrogel microparticles have a water content from 70% to 95%.

16. The composition of claim 1 further comprising one or more bioactive agents.

17. The composition of claim 1 further comprising one or more bioactive agents selected from enzymes, organic catalysts, ribozymes, organometallics, proteins, glycoproteins, peptides, polyamino acids, antibodies, nucleic acids, steroidal molecules, antibiotics, antimycotics, cytokines, growth factors, carbohydrates, oleophobics, lipids, extracellular matrix and/or its individual components, pharmaceuticals, and therapeutics.

18. The composition of claim 1 wherein:
the biodegradable unsaturated polymer is self-crosslinkable, and
the crosslinking agent is a free radical initiator.

19. The composition of claim 1 wherein:
the hydrogel microparticles comprise crosslinked collagen.

20. The composition of claim 1 wherein:
the hydrogel microparticles comprise crosslinked gelatin.

21. The composition of claim 1 wherein:
the biodegradable unsaturated polymer comprises self-crosslinkable poly(ε-caprolactone-fumarate),
the crosslinking agent is a free radical initiator, and
the composition is injectable.

22. The composition of claim 1 wherein:
the hydrogel microparticles have diameters in the range of 1 to 1000 micrometers.

23. A scaffold for tissue regeneration, the scaffold prepared by allowing any of the compositions of claims 1 to 22 to crosslink in a cavity or a mold.

24. The scaffold of claim 23 wherein:
the hydrogel microparticles comprise greater than 49% by volume and up to 99% by volume of the scaffold.

25. The scaffold of claim 24 wherein:
physiologic fluid replaces the entrapped water after the scaffold is placed into or formed in a cavity in an animal or human body.

26. A method for fabricating a scaffold for tissue regeneration, the method comprising:
allowing any of the compositions of claims 1 to 22 to crosslink in a cavity or a mold.

27. The method of claim 26 wherein:
the cavity is in an animal or human body.

28. The method of claim 27 wherein:
physiologic fluid replaces the entrapped water after the composition crosslinks in the cavity.

## Patentansprüche

1. Zusammensetzung zur Herstellung eines porösen Gerüsts, wobei die Zusammensetzung aufweist:
ein biologisch zersetzbares, ungesättigtes Polymer;
ein Vernetzungsmittel; und
einen biologisch zersetzbaren Hydrogel-Mikropartikel-Porenbildner, wobei die Hydrogel-Mikropartikel eingeschlossenes Wasser enthalten.

2. Zusammensetzung nach Anspruch 1, wobei das biologisch zersetzbare ungesättigte Polymer selbstvernetzbar ist.

3. Zusammensetzung nach Anspruch 1, wobei das Vernetzungmittel ein Freies-Radikal-Initiator ist.

4. Zusammensetzung nach Anspruch 1, wobei das Vernetzungsmittel einen Freies-Radikal-Initiator und ein Monomer zur Additionspolymerisation umfasst.

5. Zusammensetzung nach Anspruch 1, wobei die Hydrogel-Mikropartikel Kollagen, ein Kollagen-Derivativ oder Gemische hieraus umfassen.

6. Zusammensetzung nach Anspruch 1, wobei die Hydrogel-Mikropartikel vernetztes Kollagen, ein vernetztes Kollagen-Derivativ oder Gemische hieraus umfassen.

7. Zusammensetzung nach Anspruch 1, wobei die Hydrogel-Mikropartikel ein synthetisches, biologisch zersetzbares Polymer umfassen.

8. Zusammensetzung nach Anspruch 1, wobei die Hydrogel-Mikropartikel ein vernetztes synthetisches, biologisch zersetzbares Polymer umfassen.

9. Zusammensetzung nach Anspruch 1, wobei die Hydrogel-Mikropartikel oligo (Poly- (Ethylenglykol-) -Fumarat umfassen.

10. Zusammensetzung nach Anspruch 1, wobei die Hydrogel-Mikropartikel vernetztes Oligo (Poly-(Ethylenglykol-)-Fumarat) umfassen.

11. Zusammensetzung nach Anspruch 1, wobei die Hydrogel-Mikropartikel einen Wassergehalt von etwa 20 bis etwa 99 Vol.-% aufweisen.

12. Zusammensetzung nach Anspruch 1, wobei die Zusammensetzung einspritzbar ist.

13. Zusammensetzung nach Anspruch 1, wobei das biologisch zersetzbare, ungesättigte Polymer ausgewählt ist aus Poly-(Propylen-Fumarat), Poly-(ε-Caprolacton-Fumarat) und Gemischen sowie Copolymeren hiervon.

14. Zusammensetzung nach Anspruch 1, wobei das biologisch zersetzbare, ungesättigte Polymer Poly-(Propylen-Fumarat) umfasst.

15. Zusammensetzung nach Anspruch 1, wobei die Hydrogel-Mikropartikel einen Wassergehalt von 70% bis 95% aufweisen.

16. Zusammensetzung nach Anspruch 1, außerdem aufweisend ein oder mehrere bioaktive Mittel.

17. Zusammensetzung nach Anspruch 1, außerdem aufweisend ein oder mehrere bioaktive Mittel, die ausgewählt sind aus Enzymen, organischen Katalysatoren, Ribozymen, Organometallen, Proteinen, Glykoproteinen, Peptide, Polyaminosäuren, Antikörpern, Nukleinsäuren, steroidischen Molekülen, Antibiotika, Antifungiziden, Cytokinen, Wachstumsfaktoren, Carbohydraten, Oleophoben, Lipiden, extrazellularen Matrix- und/oder Einzelkomponenten, Arzneimitteln und Therapeutika.

18. Zusammensetzung nach Anspruch 1, wobei das biologisch zersetzbare, ungesättigte Polymer selbst versetzbar ist und das Vernetzungsmittel ein Freies-Radikal--Initiator ist.

19. Zusammensetzung nach Anspruch 1, wobei die Hydrogel-Mikropartikel vernetztes Kollagen umfassen.

20. Zusammensetzung nach Anspruch 1, wobei die Hydrogel-Mikropartikel vernetzte Gelatine umfassen.

21. Zusammensetzung nach Anspruch 1, wobei das biologisch zersetzbare, ungesättigte Polymer selbst vernetzbares Poly-(ε-Caprolacton-Fumarat) umfasst,
das Vernetzungsmittel ein Freies-Radikal-Initiator ist, und die Zusammensetzung einspritzbar ist.

22. Zusammensetzung nach Anspruch 1, wobei die Hydrogel-Mikropartikel Durchmesser im Bereich von 1 bis 1000 Micrometer aufweisen.

23. Gerüst zur Geweberegenerierung, wobei das Gerüst zubereitet ist, indem man jeder der Zusammensetzungen von Anspruch 1 bis 22 erlaubt, in einem Hohlraum oder einer Form sich zu vernetzen.

24. Gerüst nach Anspruch 23, wobei die Hydrogel-Mikropartikel 49 Vol.-% bis hin zu 99 Vol.-% des Gerüsts umfassen.

25. Gerüst nach Anspruch 24, wobei physiologische Flüssigkeit das eingeschlossene Wasser ersetzt, nachdem das Gerüst in einem Hohlraum in einem tierischen oder menschlichen Körper angeordnet bzw. gebildet worden ist.

26. Verfahren zur Herstellung eines Gerüsts zur Geweberegenerierung, wobei das Verfahren den Schritt umfasst:
dass man jeder der Zusammensetzungen nach Anspruch 1 bis 22 erlaubt, in einem Hohlraum oder einer Form sich zu vernetzen.

27. Verfahren nach Anspruch 26, wobei der Hohlraum ein tierischer oder menschlicher Körper ist.

28. Verfahren nach Anspruch 27, wobei physiologische Flüssigkeit das eingeschlossene Wasser ersetzt, nachdem die Zusammensetzung in dem Hohlraum vernetzt.

## Revendications

1. Composition pour fabriquer une armature poreuse, la composition comprenant :
un polymère insaturé biodégradable;
un agent de réticulation ; et
un agent porogène à base de microparticules d'hydrogel biodégradables,
dans laquelle les microparticules d'hydrogel comprennent de l'eau piégée.

2. Composition selon la revendication 1, dans laquelle:
le polymère insaturé biodégradable est auto-réticulant.

3. Composition selon la revendication 1, dans laquelle:
l'agent de réticulation est un initiateur radicalaire libre.

4. Composition selon la revendication 1, dans laquelle :
l'agent de réticulation comprend un initiateur radicalaire libre et un monomère capable de polymérisation par addition.

5. Composition selon la revendication 1, dans laquelle :
les microparticules d'hydrogel comprennent le collagène, un dérivé de collagène ou des mélanges de ceux-ci.

6. Composition selon la revendication 1, dans laquelle:
les microparticules d'hydrogel comprennent un collagène réticulé, un dérivé de collagène réticulé ou des mélanges de ceux-ci.

7. Composition selon la revendication 1, dans laquelle:
les microparticules d'hydrogel comprennent un polymère biodégradable synthétique.

8. Composition selon la revendication 1, dans laquelle :
les microparticules d'hydrogel comprennent un polymère biodégradable synthétique réticulé.

9. Composition selon la revendication 1, dans laquelle:
les microparticules d'hydrogel comprennent un oligo(fumarate de poly(éthylène glycol)).

10. Composition selon la revendication 1, dans laquelle :
les microparticules d'hydrogel comprennent un oligo(fumarate de poly(éthylène glycol)) réticulé.

11. Composition selon la revendication 1, dans laquelle :
les microparticules d'hydrogel ont une teneur en eau d'environ 20 à environ 99 % en volume.

12. Composition selon la revendication 1, dans laquelle:
la compositions est injectable.

13. Composition selon la revendication 1, dans laquelle :
le polymère insaturé biodégradable est choisi parmi le poly(fumarate de propylène), le poly(fumarate d'ε-caprolactone), leurs mélanges et leurs copolymères.

14. Composition selon la revendication 1, dans laquelle:
le polymère insaturé biodégradable comprend un poly(fumarate de propylène).

15. Composition selon la revendication 1, dans laquelle:
les microparticules d'hydrogel ont une teneur en eau de 70 à 95 %.

16. Composition selon la revendication 1 comprenant, en outre, un ou plusieurs agents bioactifs.

17. Composition selon la revendication 1 comprenant, en outre, un ou plusieurs agents bioactifs choisis parmi les enzymes, les catalyseurs organiques, les ribozymes, les organométalliques, les protéines, les glycoprotéines, les peptides, les acides polyaminés, les anticorps, les acides nucléiques, les molécules stéroïdiennes, les antibiotiques, les antimycotiques, les cytokines, les facteurs de croissance, les glucides, les oléophobes, les lipides, une matrice extracellulaire et/ou ses composants individuels, les composés pharmaceutiques et les agents thérapeutiques.

18. Composition selon la revendication 1, dans laquelle :
le polymère insaturé biodégradable est auto-réticulant, et
l'agent de réticulation est un initiateur radicalaire libre.

19. Composition selon la revendication 1, dans laquelle:
les microparticules d'hydrogel comprennent un collagène réticulé.

20. Composition selon la revendication 1, dans laquelle :
les microparticules d'hydrogel comprennent une gélatine réticulée.

21. Composition selon la revendication 1, dans laquelle :
le polymère insaturé biodégradable comprend un poly(fumarate d'ε-caprolactone) auto-réticulant,
l'agent de réticulation est un initiateur radicalaire libre, et
la composition est injectable.

22. Composition selon la revendication 1, dans laquelle:
les microparticules d'hydrogel ont des diamètres dans la plage de 1 à 1000 micromètres.

23. Armature pour régénération tissulaire, l'armature étant préparée en laissant l'une quelconque des compositions selon les revendications 1 à 22 réticuler dans une cavité ou un moule.

24. Armature selon la revendication 23, dans laquelle:
les microparticules d'hydrogel représentent plus de 49 % en volume et jusqu'à 99 % en volume de l'armature.

25. Armature selon la revendication 24, dans laquelle :
un fluide physiologique remplace l'eau piégée après que l'armature est placée, ou formée dans une cavité d'un corps animal ou humain.

26. Procédé de fabrication d'une armature pour régénération tissulaire, le procédé comprenant l'étape consistant à :
laisser l'une quelconque des compositions selon les revendications 1 à 22 réticuler dans une cavité ou un moule.

27. Procédé selon la revendication 26, dans lequel :
la cavité fait partie d'un corps animal ou humain.

28. Procédé selon la revendication 27, dans lequel:
un fluide physiologique remplace l'eau piégée après que la composition a réticulé dans la cavité.
